# EUROPÄISCHE PATENTSCHRIFT

(11) **EP 2 985 350 B1**
(45) Veröffentlichungstag und Bekanntmachung des Hinweises auf die Patenterteilung: **04.10.2017**
(21) Anmeldenummer: 14181074.7
(22) Anmeldetag: 14.08.2014
(51) Int. Cl.: C12Q 1/68

(54) **Verfahren zur Mikrobiom-Analyse**
Method for microbiome analysis
Procédé d'analyse de microbiome

(43) Veröffentlichungstag der Anmeldung: 17.02.2016
(73) Patentinhaber: microBIOMix GmbH, 93053 Regensburg (DE)
(72) Erfinder: REISCHL, Udo, 93107 Thalmassing (DE); GESSNER, André, 93059 Regensburg (DE); HIERGEIST, Andreas, 93051 Regensburg (DE)
(74) Vertreter: Hannke, Christian

(56) Entgegenhaltungen:
- WO-A2-2004/055205
- MUMY K L ET AL: "CONVENIENT DETERMINATION OF DNA EXTRACTION EFFICIENCY USING AN EXTERNAL DNA RECOVERY STANDARD AND QUANTITATIVE-COMPETITIVE PCR", JOURNAL OF MICROBIOLOGICAL METHODS, ELSEVIER, AMSTERDAM, NL, Bd. 57, Nr. 2, 14. März 2004 (2004-03-14), Seiten 259-268, XP002436310, ISSN: 0167-7012, DOI: 10.1016/J.MIMET.2004.01.013
- Y. PAN ET AL: "Impacts of Inter- and Intralaboratory Variations on the Reproducibility of Microbial Community Analyses", APPLIED AND ENVIRONMENTAL MICROBIOLOGY, Bd. 76, Nr. 22, 24. September 2010 (2010-09-24), Seiten 7451-7458, XP055182990, ISSN: 0099-2240, DOI: 10.1128/AEM.01595-10
- GISKE BIESBROEK ET AL: "Deep Sequencing Analyses of Low Density Microbial Communities: Working at the Boundary of Accurate Microbiota Detection", PLOS ONE, Bd. 7, Nr. 3, 6. März 2012 (2012-03-06), Seite e32942, XP055183102, DOI: 10.1371/journal.pone.0032942
- A. K. BARTRAM ET AL: "Generation of Multimillion-Sequence 16S rRNA Gene Libraries from Complex Microbial Communities by Assembling Paired-End Illumina Reads", APPLIED AND ENVIRONMENTAL MICROBIOLOGY, Bd. 77, Nr. 11, 1. April 2011 (2011-04-01) , Seiten 3846-3852, XP055182941, ISSN: 0099-2240, DOI: 10.1128/AEM.02772-10
- PERNILLE JOHANSEN ET AL: "Development of quantitative PCR and metagenomics-based approaches for strain quantification of a defined mixed-strain starter culture", SYSTEMATIC AND APPLIED MICROBIOLOGY, Bd. 37, Nr. 3, 28. Februar 2014 (2014-02-28), Seiten 186-193, XP055128854, ISSN: 0723-2020, DOI: 10.1016/j.syapm.2013.12.006

## Beschreibung

Die vorliegende Erfindung betrifft ein Verfahren zur Mikrobiom-Analyse unter Verwendung eines internen Standards.

Außerdem ist die Verwendung eines Kits zur Durchführung dieses Verfahrens Gegenstand der Erfindung. Ein solches Kit umfasst einen internen Standard, der zur Mikrobiom-Analyse eingesetzt werden kann. Als Mikrobiom wird allgemein die Gesamtheit der den (menschlichen oder tierischen) Körper besiedelnden Mikroorganismen bezeichnet. Im Folgenden wird der Erfindungsgegenstand am Beispiel des Mikrobioms des menschlichen Körpers beschrieben, jedoch ist auch denkbar, dass das Verfahren auch zur Analyse anderer Populationen von Mikroorganismen eingesetzt werden kann.

Aus dem Stand der Technik bekannte Schätzungen gehen davon aus, dass etwa 10¹⁴ mikrobielle Zellen einen menschlichen Körper besiedeln. Diese Zahl übersteigt damit die Anzahl humaner Zellen um mehr als das zehnfache. Die Erbinformation der einen menschlichen Körper besiedelnden Mikrobiota übersteigt die humane Erbinformation schätzungsweise um etwa ein 150-faches. Da Veränderungen in der Zusammensetzung des Mikrobioms beim Menschen mit einer Vielzahl von physiologischen Veränderungen und somit auch der Entstehung unterschiedlicher Krankheitsbilder (z.B. Adipositas, Allergien, chronisch-entzündliche Darmerkrankungen, Diabetes, neurologische Erkrankungen) in Verbindung gebracht werden (Hofer et al., 2014; Kostic et al., 2014; Moloney et. al. 2014; Tremaroli et al, 2012), ist eine Analyse des Mikrobioms in den Fokus vieler Forschungsdisziplinen gelangt. Insbesondere nach der vollständigen Entschlüsselung des menschlichen Genoms (Human Genome Project) wurden Bestrebungen unternommen, auch das Genom möglichst aller den menschlichen Körper besiedelnder Mikrobiota zu entschlüsseln.

Die Entwicklung moderner Hochdurchsatz-Nukleinsäuresequenziertechnologien (z.B. sogenannte "Next Generation Sequencing"-Technologien) ermöglichte die kulturunabhängige Analyse des Metagenoms (also der Gesamtheit der in einem Habitat vorhandenen genetischen Information). Eine Analyse der Zusammensetzung des Mikrobioms mit klassischen mikrobiologischen Kulturverfahren war zuvor nicht umfassend möglich, da schätzungsweise mehr als 80 % der Mikrobiota nicht unter derzeit verfügbaren Laborbedingungen kultivierbar sind. Die nun verfügbaren Hochdurchsatz-Nukleinsäuresequenziertechnologien ermöglichen darüber hinaus die Identifizierung und relative Quantifizierung der Mitglieder mikrobieller Gemeinschaften. Dadurch konnten Ansätze des Verständnisses für die fundamentale Bedeutung des Mikrobioms für den (menschlichen) Körper und dessen Zusammenspiel mit physiologischen Prozessen wie beispielsweise der engen Verknüpfung mit dem Immunsystem (Belkaid et al. 2014) oder neurologischen Prozessen (Foster et al., 2013) geschaffen werden.

Aus dem Stand der Technik sind molekulargenetischen Analysen des Mikrobioms bekannt, die auf einem mehrstufigen Prozess basieren. Diese Analysen basieren im Wesentlichen entweder einerseits auf Amplicon-basierten Sequenz-Analysen von informationsreichen Segmenten oder den kompletten universellen Markergenen (z.B. 16S rDNA), die durch eine PCR amplifiziert und anschließend mit unterschiedlichen Sequenzier-Technologien (z.B. Next Generation Sequencing Technologien) analysiert werden oder andererseits auf der direkten Analyse des gesamten genetischen Information durch sogenannte Metagenom (oder auch "Shotgun") Analysen.

Gemeinsam haben beide Arten der Analyse, dass im ersten Schritt die Isolierung der DNA aus der entsprechenden Probenmatrix (z.B. Stuhlproben, Biopsien, Abstriche) notwendig ist. Aus dem Stand der Technik sind dafür kommerziell verfügbare DNA-Isolierungskits oder nicht standardisierte manuelle in-house Methoden bekannt. Eine effiziente und gleichförmige Isolierung der Gesamtnukleinsäure aus bestimmten komplexen Probenmaterialien (z.B. Stuhl, Lebensmittel oder Umweltproben) ist aus enzymatischen und/oder biophysikalischen Gründen sehr problematisch. Abweichungen von der ideal gleichförmigen Isolierung der Gesamtnukleinsäure führen zwangsläufig zu einer nicht standardisierbaren oder vorhersehbaren Ungenauigkeit der Verteilung der in der resultierenden DNA-Präparation repräsentierten Bakterienspezies im Vergleich zu derjenigen in dem komplexen Probenmaterialien. Anschließend wird eine PCR-Amplifikation von Markergenen mit marktüblichen DNA-Polymerasen durchgeführt und die Nukleinsäuresequenzen der Amplifikate in hochparalleler Weise mit Next Generation Sequencing Technologien bestimmt. Die Gesamtheit der einzelnen unabhängig voneinander aus dem zu untersuchenden Probenmaterial ermittelten (i.d.R. mehrerer hunderttausender Einzelsequenzen) Nukleinsäuresequenzen einer Probe wird anschließend mit bioinformatorischen Mitteln (z.B. durch die öffentlich verfügbaren Open Source Softwarepakete QIIME und mothur) ausgewertet.

Diese Auswertung beinhaltet die Qualitätskontrolle der Sequenzdaten sowie die Reduktion typischer, plattformabhängiger Sequenzierfehler. Anschließend werden ähnliche Sequenzen (z.B. > 97 % Nukleinsäuresequenz-Identität) zu sogenannten OTUs (Operational Taxonomic Units) zusammengefasst (geclustert) und jedes OTU durch Alignment mit bekannten Nukleotidsequenzen verfügbarer Datenbanken (z.B. 16S rDNA-Sequenzdatenbanken: Silva, Greengenes, Ribosomal Database Project oder Chaperonin-Datenbank cpnDB) abgeglichen. Die Sequenzen werden anschließend entsprechend der aktuellen Taxonomie zugeordnet und phylogenetisch analysiert. Die sich aus den Analysen ergebenden Taxa werden gemäß ihrer relativen Häufigkeit im analysierten Sequenzdatensatz dargestellt.

Zum Vergleich der Diversität einzelner Proben oder Probengruppen finden Methoden der multivarianten Statistik Anwendung. Aus Veränderungen der relativen Häufigkeit einzelner oder mehrerer Taxa oder der Veränderung der abgeleiteten mikrobiellen Diversität (alpha- und beta-Diversität) in der Probe wird versucht, einen Zusammenhang zu klinischen Bildern herzustellen.

Mumy KL et al.; Convenient determination of DNA extraction efficiency using an external DNA recovery standard and quantitative-competitive PCR. J Microbiol Methods. 2004 May; 57(2): 259-68, beschreibt ein Verfahren zum Vergleich verschiedener DNA-Extraktions-Kits. Durchgeführt wird der Vergleich am Beispiel einer Bodenprobe durch q-PCR, wodurch eine Quantifizierung der in der Bodenprobe enthaltenen und aufgeschlossenen Bakterien-DNA möglich wird. Dazu wird einer Bodenprobe ein externer DNA-Standard zugesetzt. Dieser ist artifiziell und derart modifiziert, dass er von natürlicher bakterieller DNA unterscheidbar ist. Der Standard enthält ein 500 bp-Fragment der Bakteriophage Lambda das in pBR322 eingefügt und in *e. coli* transformiert wurde.

Pan Y et al.; Impacts of inter- and intralaboratory variations on the reproducibility of microbial community analyses. Appl Environ Microbiol. 2010 Nov; 76(22):7451-8, beschreibt den Vergleich der Ergebnisse der Analyse einer Bodenprobe (eines italienischen Reisfelds) durch verschiedene Analysenlabore, die jeweils dasselbe Protokoll verwenden. Ziel dieser Untersuchung ist es, Aussagen über die Vergleichbarkeit derartiger Analysen durch unterschiedliche Labore treffen zu können. Als zu quantifizierende DNA wurde DNA einer Vielzahl von Methanoxidierender Bakterien (MOB) ausgewählt.

Aus Biesbroek G et al. (2012) Deep Sequencing Analyses of Low Density Microbial Communities: Working at the Boundary of Accurate Microbiota Detection. PLoS ONE 7(3): e32942, sind Untersuchungen zur Analyse der Zusammensetzung der Mikrobiota in Proben geringer Bakteriendichte beschrieben. Die Quantifizierung der in der Probe enthaltenen DNA erfolgt mittels quantitativer PCR. Weiterhin ist beschrieben, dass die Gesamtbakterienzahl mittels quantitativer PCR unter Verwendung universeller Primer, die zur Amplifikation des 16S rDNA-Gen geeignet sind, bestimmt wurde.

Aus der Druckschrift Bartram AK et al.; Generation of multimillion-sequence 16S rRNA gene libraries from complex microbial communities by assembling paired-end illumina reads. Appl Environ Microbiol. 2011 Jun;77(11):3846-52, ist ein Gemisch von DNA verschiedener Herkunft bekannt, mit dem die Fähigkeit verschiedener Geräte/Verfahren zur parallelen Sequenzierung einer Vielzahl von Genen überprüft werden kann.

Diese aus dem Stand der Technik bekannten Verfahren weisen erhebliche Nachteile auf, wie sie beispielsweise von Klindworth et al., 2013; Mao et al., 2012; Pilloni et al., 2012 und von Wintzingerode et al, 1997 dargestellt wurden. Die Nachteile resultieren beispielsweise aus den zur DNA-Isolierung aus der Proben-Matrix verwendeten Verfahren, der Auswahl der PCR-Primer zur Amplifizierung von bestimmten Segmenten des Markergens, der Auswahl der amplifizierten Region des Markergens, der Auswahl der thermostabilen DNA-Polymerase (beispielsweise Enzyme mit oder ohne proofreading Eigenschaft, Enzyme mit unterschiedlicher Effizienz für die Amplifikation von längeren Sequenzabschnitten oder "problematischen" Sequenzmotiven innerhalb der Amplifikationsprodukte), der Auswahl der PCR-Bedingungen und der Auswahl der Sequenzierungsmethode für die Nukleinsäuren, da alle diese Faktoren eine nicht vorhersehbare Auswirkung (z.B. Schwankungen in der Zusammensetzung der detektierten Nukleinsäuren) auf die abschließende Analyse der Zusammensetzung des Mikrobioms haben können.

Insbesondere eine uniforme Zell-Lyse der in der Probe vorhandenen Mikroorganismen, die sich in ihrer Zellwandstruktur und somit ihrer Lysefähigkeit unterscheiden, ist bei der Analyse der Zusammensetzung mikrobieller Gemeinschaften wichtig. Eine möglichst vollständige und reproduzierbare DNA-Präparation aus derartigen teilweise äußerst heterogenen Proben ist entscheidend für den Erfolg bzw. die Zuverlässigkeit aller anschließenden Prozesse zur Ermittlung des tatsächlichen Mikrobioms, bzw. Metagenoms im Untersuchungsmaterial. Dies betrifft sowohl die Art der vorhandenen bakteriellen Spezies mit ihren speziesspezifischen Eigenheiten in Zellwandstruktur oder biophysikalischer Robustheit als auch die Effizienz der Nukleinsäureextraktion selbst. Es ist bekannt, dass in Abhängigkeit von der gewählten Lysemethode, die Verteilung der isolierten DNA aufgrund beispielsweise unterschiedlicher Rigidität der Zellwand stark variieren kann. Dies führt wiederum, zwangsläufig zu einer (nachträglich nicht quantifizierbaren oder erkennbaren) Unter- bzw. Überrepräsentierung verschiedener Spezies in den folgenden Prozessschritten und der anschließenden Nukleotidsequenzanalyse.

Eine weitere bedeutende Ursache für Verfälschungen der detektierten Mikrobiomzusammensetzung gegenüber der wirklich vorliegenden Mikrobiomzusammensetzung resultiert aus der PCR-Amplifikation und der Analyse und Zuordnung der Amplifizierten DNA. Die Zuordnung von Nukleinsäure-Sequenzabschnitten universeller Markergene erfolgt auf Grundlage der Sequenzvariabilität in unterschiedlichen Regionen des amplifizierten DNA-Fragments. Die PCR-Amplifikation dieser universellen Markergene aus dem zuvor gewonnenen Nukleinsäureextrakt kann durch nukleinsäuresequenzabhängige Unregelmäßigkeiten in der Amplifikation (z.B. durch schlechtere Amplifizierbarkeit G/C- oder A/T-reicher Sequenzabschnitte durch die verwendete Art der DNA-Polymerase) dieser variablen Abschnitte ebenfalls zu einer Verschiebung der relativen Häufigkeit der beobachteten Taxa und damit zu einer fehlerhaften Repräsentation der Zusammensetzung des Mikrobioms in der untersuchten Probe führen.
Die aus dem Stand der Technik bekannte Analyse der Sequenzdaten und die daraus resultierende Berechnung von relativen Häufigkeiten einzelner Taxa in den Sequenzdaten kann zu Fehlinterpretationen der Veränderung der absoluten Anzahl einzelner oder mehrerer Taxa in den Mikrobiom-Daten führen. So ist beispielsweise nicht unterscheidbar, ob die Gesamtzahl aller Bakterien in der Probe abgenommen oder die Anzahl von Bakterien eines einzelnen Taxons zugenommen hat. Die relative Häufigkeit würde in beiden Fällen möglicherweise gleich bleiben und deshalb die tatsächliche Veränderung im Mikrobiom nur unzureichend nachvollziehbar wiedergeben.

Diese bekannten Nachteile der aus dem Stand der Technik bekannten Verfahren machen eine Prozesskontrolle notwendig mit der sich die Auswirkungen dieser Nachteile nachvollziehen lassen und es während der Datenanalyse ermöglicht, entsprechenden Korrekturen durchzuführen.

Aufgabe der vorliegenden Erfindung ist es daher, ein Verfahren bereitzustellen, das eine absolute Quantifizierung der Bakterien in einer Probe ermöglicht. Weiterhin ist es Aufgabe der Erfindung, eine Vorrichtung bereit zu stellen, die eine quantifizierbare Mikrobiomanalyse ermöglicht. Außerdem ist es Aufgabe der Erfindung, ein Kit bereit zu stellen, mittels welchem prozessbedingte Schwankungen in der Aufbereitung, Amplifizierung und Analyse Verteilung der einen Körper besiedelnden Mikrobiota detektiert werden können.

Gelöst wird diese Aufgabe durch ein Verfahren zur Mikrobiom-Analyse nach Anspruch 1, wobei einer Probe ein quantifizierbarer Standard zugesetzt wird, der Spike-Bakterien umfasst, die mindestens zwei Bakterienspezies umfassen, und die Probe anschließend gemeinsam mit dem Standard analysiert wird.

Außerdem wird die Aufgabe durch ein Kit zur Durchführung eines solchen Verfahrens gelöst, das einen quantifizierbaren Standard für eine Mikrobiom-Analyse, der Spike-Bakterien umfasst, die mindestens zwei Bakterienspezies umfassen, und eine Anweisung zur Durchführung einer Mikrobiom-Analyse umfasst.

Bei den Spike-Bakterien handelt es sich bevorzug um ein Bakterienisolat oder eine Mischung verschiedener Bakterienisolate bzw. Bakterienspezies. Die Begriffe Spike-Bakterien und Bakterienisolat bzw. Bakterien-Isolat werden daher im Folgenden synonym verwendet. Generell sollen unter Spike-Bakterien exogene Bakterien oder Bakterienspezies verstanden werden, die einer Probe zugesetzt werden.

Durch das Verfahren zur Mikrobiom-Analyse, wobei einer Probe ein quantifizierbarer Standard zugesetzt wird, der Spike-Bakterien umfasst, die mindestens zwei Bakterienspezies umfassen, und die Probe anschließend gemeinsam mit dem Standard analysiert wird, können die aus dem Stand der Technik bekannten Nachteile ausgeräumt werden. Bei einer Einführung eines multifunktionellen internen Standards für Mikrobiom-Analysen, kann dieser sowohl als Prozesskontrolle als auch zur Normalisierung der Sequenzdaten dienen.

Erfindungsgemäß umfasst die Mikrobiomanalyse mindestens die Schritte Amplifikation von universellen Markergenen, Nukleinsäuresequenzierung, Sequenzanalyse und mindestens einen Quantifizierungsschritt mittels quantitativer Echtzeit-PCR. Bei der Mikrobiomanalyse handelt es sich somit um einen mehrstufigen Prozess. Bevorzugt wird der quantifizierbarer Standard in einem sehr frühen Prozessschritt zugesetzt und über alle Prozessschritte (bevorzugt mindestens bis zur Nukleinsäuresequenzierung) genau wie die übrige Probe behandelt. Bevorzugt wird der quantifizierbare Standard der Probe vor der DNA-Isolierung zugesetzt.

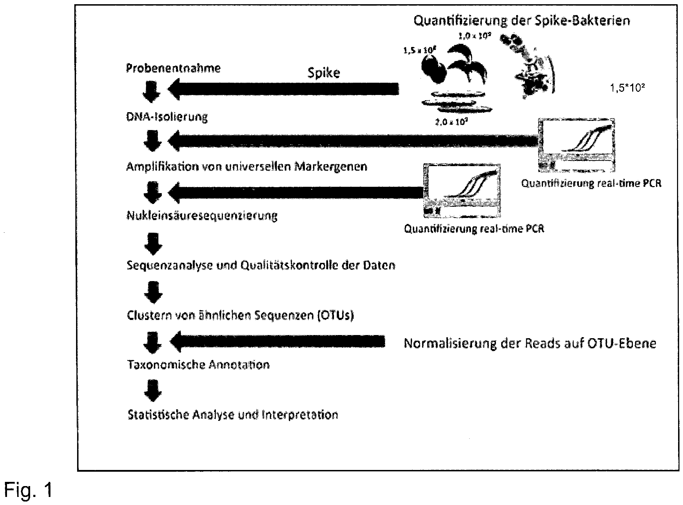

Die Mikrobiomanalyse umfasst bevorzugt weiterhin Schritte, die ausgewählt sind aus der Gruppe bestehend aus Probenentnahme, DNA-Isolierung, Sequenzanalyse, Qualitätskontrolle, Clustern von ähnlichen Sequenzen, taxonomische Annotation, statistische Analyse und Interpretation der ermittelten Daten. Auch wenn eine Mikrobiomanalyse üblicherweise von einer Probenentnahme ausgeht und wie in Fig. 1 dargestellt über DNA-Isolierung, Amplifikation von universellen Markergenen, Nukleinsäuresequenzierung, Sequenzanalyse, Qualitätskontrolle, Clustern von ähnlichen Sequenzen, taxonomische Annotation und statistische Analyse und Interpretation der ermittelten Daten alle oben genannten Schritte umfasst, soll im Folgenden als Mikrobiomanalyse auch ein Verfahren bezeichnet werden, dass einen oder einige der genannten Prozessschritte nicht aufweist. Beispielweise kann der Prozessschritt der Probenentnahme zeitlich und/oder örtlich versetzt erfolgen. Dennoch soll von der Bezeichnung Mikrobiomanalyse auch ein Verfahren eingeschlossen sein, bei dem nur die Analyse einer Probe erfolgt, weil die Probeentnahme beispielsweise von einem Arzt vorgenommen wurde und die Probe an ein Analyselabor versendet wird. Außerdem soll durch die vorliegende Erfindung auch ein Verfahren eingeschlossen sein, bei dem beispielsweise die Auswertung der Rohdaten oder auch lediglich Teile der Auswertung zeitlich und/oder örtlich versetzt von den übrigen Verfahrensschritten erfolgen.

Der der Probe zugegebene (bevorzugt) exogene Standard umfasst bevorzugt Spike-Bakterien, die mindestens 2, bevorzugt mindestens 3, weiter bevorzugt mindestens 5, weiter bevorzugt mindestens 10 verschiedene Bakterienspezies umfassen. Diese werden vorteilhafterweise in quantifizierbarer Anzahl der Probenmatrix zugeführt. Diese Bakterien werden bevorzugt zusammen mit der Mikrobiota im ausgewählten DNA-Isolierungsverfahren lysiert und die Gesamtheit der vorhandenen Nukleinsäuren isoliert.

Bevorzugt werden in einem folgenden Prozessschritt des Verfahrens durch Auswahl universeller PCR-Primer Markergen-Regionen der gesamten Mikrobiota der Mischung aus Probe und Standard mittels quantitativer Echtzeit-PCR amplifiziert und bevorzugt anschließend die Kopienzahl der Markergene im resultierenden PCR-Amplifikationsgemisch, und besonders bevorzugt auch in der ursprünglichen Probe nach der Isolierung der DNA, quantitativ bestimmt. Demnach werden durch universelle PCR-Primer ausgewählte Markergen-Regionen der gesamten Mikrobiota (incl. der Spike-Kontroll- Bakterien) durch universelle PCR-Primer in einer PCR-Reaktion amplifiziert.

Weiter bevorzugt ist ein Verfahren, bei dem einer quantitativen Echtzeit-PCR ein externer Quantifizierungsstandard und universelle Primerpaare zugesetzt werden und die Gesamtzahl an Markergenkopien in der Mischung aus Probe und Standards durch absolute Quantifizierung bestimmt wird. Durch quantitative real-time PCRs lässt sich die Kopienzahl der Markergene nach der Isolierung der DNA und im resultierenden PCR-Amplifikationsgemisch quantitativ bestimmen. Zusätzlich kann die Gesamtzahl an Markergenkopien in der gesamten Probe (incl. Spike-Kontrollbakterien) durch absolute Quantifizierung anhand eines externen Standards mit Hilfe einer quantitativen real-time PCR und universellen Primerpaaren bestimmt werden. So ist eine zusätzliche Kontrolle der relativen Häufigkeiten der Spike-Kontrolle in der aufbereiteten Probe möglich und ein Vergleich mit der Konstellation in den Sequenzdaten möglich. Eine Veränderung der Anzahl der quantifizierten Markergenkopien für ein oder mehrere Kontroll-Spike-Bakterien während des Prozesses deutet auf einen methodisch bedingte Varianz der Probenvorbereitung (z.B. durch ungleichmäßigen Lyse unterschiedlicher Bakterien, nicht homogene Amplifikation der Markergene oder inhibitorische Effekte während der Probenaufarbeitung) hin.
Ursachen für eine methodisch bedingte Varianz können verschiedene Ursachen haben. Um möglichst viele dieser Ursachen ausschließen zu können, ist in einer weiteren bevorzugten Variante des Verfahrens vorgesehen, dass mindestens eine Bakterienspezies der Spike-Bakterien mindestens 2, bevorzugt mindestens 3, bevorzugt mindestens 4, weiter bevorzugt mindestens 5, weiter bevorzugt mindestens 6, besonders bevorzugt alle der Eigenschaften aus einer Gruppe von Eigenschaften aufweist, die die Eigenschaften
- Nukleinsäuresequenzabschnitte eines Markergens der Bakterienspezies weisen weniger als 95 % Sequenzidentität mit den in verfügbaren Humanmikrobiom-Sequenzdatenbanken hinterlegten Nukleinsäuresequenzen auf,
- die Bakterienspezies gehört nicht einem Genus an, dessen Vorkommen im Human- und/oder Tier-Mikrobiom beschrieben ist,
- die Bakterienspezies, bevorzugt die Spike-Bakterien, ist/sind unter verfügbaren mikrobiologischen Methoden im Labor kultivierbar,
- die Bakterienspezies, bevorzugt die Spike-Bakterien, ist/sind mit mikroskopischen Methoden in wässriger Lösung quantifizierbar,
- die Speziesmarker-Gen Kopien der Bakterienspezies der Spike-Bakterien sind durch eine spezifische quantitative Echtzeit-PCR quantifizierbar, bevorzugt auch in komplexen Gengemischen, besonders bevorzug auch in komplexen Spike-Bakterien-Mischungen,
- die Bakterienspezies gehört einem Phylum an, der im humanen Mikrobiom ebenfalls vertreten ist, wobei bei mehreren Bakterienspezies der Spike-Bakterien mindestens zwei der Bakterienspezies zu unterschiedlichen Phyla gehören,
- die intragenomische Anzahl der Speziesmarker-Gene der Bakterien der Spike-Bakterien ist bekannt und kann beim Prozess der Quantifizierung berücksichtigt werden,
umfasst. Falls die Spike-Bakterien mehrere Bakterienspezies umfassen ist bevorzugter Weise weiterhin vorgesehen, dass mindestens zwei Bakterienspezies unterschiedliche Zellwandstrukturen besitzen (z.B. Gram-positiv, Gram-negativ) und/oder mindestens zwei Bakterienspezies auswählbare Speziesmarker-Gene mit unterschiedlichem G/C-Gehalt aufweisen.

Weiter bevorzugt ist ein Verfahren, bei dem mindestens eine Bakterienspezies der Spike-Bakterien, bevorzugt mindestens zwei Bakterienspezies der Spike-Bakterien, weiter bevorzugt mindestens drei Bakterienspezies der Spike-Bakterien, besonders bevorzugt mindestens fünf Bakterienspezies der Spike-Bakterien aus einer Gruppe ausgewählt sind, die die Bakterienspezies *Salinibacter ruber, Alicyclobacillus acidiphilus, Rhizobium radiobacter, Sphaerobacter thermophilus, Thermomicrobium roseum, Thiomicrospira arctica, Acidimicrobium ferrooxidans, Alcanivorax borkumensis, Colwellia psychrerythraea, Idiomarina loihiensis, Marinilabilia salmonicolor, Thioalkalibacter halophilus, Rhodobaca bogoriensis, Sulfurimonas autotrophica, Arcobacter halophilus, Aquisalibacillus elongates, Haloactinobacterium album, Geobacillus stearothermophilus* und *Geobacillus caldoxylolyticus* umfasst.

Diese Bakterienspezies alleine oder in Kombination mit anderen erfüllen die oben genannten Kriterien. Selbstverständlich ist diese Aufzählung nur exemplarisch und die Erfindung ist ausdrücklich nicht auf die Verwendung dieser Bakterienspezies beschränkt.

Die Auswahl der Bakterien sollte möglichst vielfältig sein um einerseits die quantitative Kontrolle der Bakterien-Lyse und PCR-Amplifikation der Markergene der ausgewählten Bakterien zu ermöglichen und andererseits auch möglichst viele der oben genannten Kriterien zu erfüllen, um so wiederum die Vielfalt der Bakterien im humanen Mikrobiom in Bezug auf Zellwandstruktur, phylogenetischer Vergleichbarkeit und Sequenzvariabilität repräsentieren zu können.

Die quantitative Bestimmung der Markergen-Kopienzahl der Kontroll-Bakterien in der Probe ermöglicht zusätzlich eine Normalisierung der gewonnenen Sequenzdaten der Mikrobiota. Daher ist in einer weiteren bevorzugten Variante des Verfahrens vorgesehen, dass eine Normalisierung der absoluten Menge an DNA-Sequenzen eines Clusters ähnlicher Sequenzen (OTUs) anhand der Read-Anzahl an Sequenzen eines oder mehrere Bakterien des Standards erfolgt. Die Quotienten der Read-Anzahl von weiteren quantifizierten und exogen zugegebenen Bakterien dienen hierbei zusätzlich als Kontrolle der Read-Normalisierung.

Weitere Vorteile und Zweckmäßigkeiten sind exemplarisch anhand der nachfolgenden Beschreibung einer bevorzugten Ausführungsform in Verbindung mit den Figuren zu entnehmen. Hierbei zeigen:
- Fig. 1: ein Ablaufschema zur standardisierten Mikrobiom-Analyse durch Zugaben eines externen Standards;
- Fig. 2: eine beispielhafte Amplifikationskurve einer quantitativen Echtzeit-PCR zur Quantifizierung der 16S rDNA-Kopienzahl nach Zusatz eines externen Standards;
- Fig. 3: eine grafische Darstellung der durch quantitative Echtzeit-PCR bestimmten Kopienzahl von 16S rDNA;
- Fig. 4: eine beispielhafte Amplifikationskurve einer quantitativen Echtzeit-PCR zur Quantifizierung der gesamten 16S rDNA-Kopienzahl nach Zusatz eines externen Standards unter Verwendung universeller Primer;
- Fig. 5: eine grafische Darstellung der durch quantitative Echtzeit-PCR bestimmten gesamten 16S rDNA-Kopienzahl;
- Fig. 6: Abbildung einer Agarosegels nach Elektrophorese der 16S rDNA-Amplifikate;
- Fig. 7: eine graphische Darstellung des Ergebnisses der Analyse der SequenzRohdaten;
- Fig. 8: eine beispielhafte Darstellung der relativen Häufigkeit von taxonomisch annotierten OTUs in 454 GS Junior Sequenzdaten für zwei ausgewählte Proben; und
- Fig. 9a - 9c: die Verteilung der in der Mikrobiom-Analyse erhaltenen Reads je zugeordneter taxonomischen Einheiten (OTUs).

Figur 1 zeigt ein beispielhaftes Ablaufschema für eine standardisierte Mikrobiom-Analyse durch sogenannte "Spike-In"-Kontrollen. Zunächst werden dazu die "Spike-In"-Bakterien vorbereitet. Diese werden unter Laborbedingungen kultiviert und anschließend quantifiziert. Je nach Bedarf werden verschiedene Bakterienspezies zusammen kultiviert oder nach der Kultivierung zu einer Mischung eines Standards für die Mikrobiom-Analyse vereinigt. Wie oben beschrieben kann ein solcher Standard eine bis etwa 10 oder sogar mehr verschiedenen Bakterienspezies umfassen.

Ein solcher quantifizierter Standard wird wie schematisch dargestellt einer Probe zugesetzt. Diese Probe wird einem Individuum auf geeignete Weise entnommen. Bei einer solchen Probe kann es sich beispielsweise um eine Stuhlprobe, einen Abstrich oder ähnliches handeln. Gemeinsam mit den Bakterien des Standards werden die in der Probe enthaltenen Bakterien nun weiterbehandelt. Zunächst erfolgt die Lyse der Zellen. Hierfür können bekannte Verfahren verwendet werden. Ein großer Vorteil eines möglichst variantenreichen Standards mit Bakterienspezies mit verschiedenen Zellwandeigenschaften ist, dass auch durch das Lyseverfahren bedingte Verschiebungen in der Häufigkeit der DNA-Sequenzen verschiedener Bakterienspezies durch Normalisierung ausgeglichen werden können.

Nach der Lyse wird die DNA isoliert. Auch hierfür sind Verfahren und Geräte allgemein bekannt. Beispielsweise kann eine standardisierte parallele "NGS-grade" DNA-Isolierung mit Geräten wie Roche MagNA Pure 96 oder Roche MagNA Pure Compact erfolgen.

Die so isolierte DNA kann anschließend amplifiziert werden. Je nach Auswahl der Primer kann die Amplifikation auf universelle Markergene eingeschränkt werden. Sowohl vor als auch nach der Amplifikation der gesamten Probe einschließlich der DNA der Spike-Bakterien kann einen zusätzliche Quantifizierung der Spike Bakterien mittels einer quantitativen Echtzeit-PCR erfolgen. Ein Vergleich dieser Quantifizierungen kann Hinweise darauf liefern, ob Unregelmäßigkeiten während der Amplifikation der gesamten Probe aufgetreten sind. Die quantitative Echtzeit-PCR bietet den Vorteil, dass durch die bekannte Menge an Standard die Quantifizierung sehr leicht möglich ist. Außerdem bietet sie ausgezeichnete Möglichkeiten der Prozesskontrolle. Auch die Amplifikation der gesamten Probe einschließlich der Spike-Bakterien kann mittels quantitativer Echtzeit-PCR erfolgen.

Anschließend an die Amplifikation kann die Sequenzierung erfolgen. Zur Sequenzierung der DNA werden bevorzugt Hochdurchsatz (high-Throughput)-Geräte eingesetzt. Wie oben erwähnt bietet das sogenannte Next Generation Sequencing enorme Vorteile in Bezug auf die Geschwindigkeit und die Genauigkeit der ermittelten Sequenzdaten. Als ein mögliches Gerät zur Sequenzanalyse hat sich das Roche GS FLX+ erwiesen.

Nach dem Ermitteln der DNA-Sequenzen erfolgt eine Sequenzanalyse und eine erste Qualitätskontrolle der Daten. Anschließend werden ähnliche Sequenzen zu Clustern ähnlicher Sequenzen (OTUs) zusammengefasst. Anschließend erfolgt eine Normalisierung der absoluten Menge an DNA-Sequenzen eines OTU anhand der Read-Anzahl an Sequenzen von Bakterien des Standards. Die Quotienten der Read-Anzahl von weiteren quantifizierten und exogen zugegebenen Bakterien können hierbei zusätzlich als Kontrolle der Read-Normalisierung dienen.

Auf Basis der so ermittelten Ergebnisse kann eine taxonomische Annotation erfolgen, die dann schließlich die statistische Analyse und Interpretation ermöglicht.

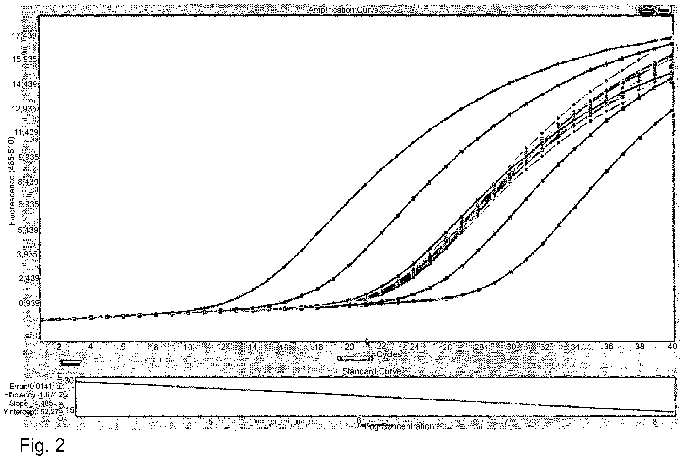

In den Figuren 2 - 9 sind Ergebnisse einer Mikrobiomanalyse mit einem Standard aus drei definierten Spike-Bakterien zur Analyse des Darmmikrobioms der Maus dargestellt. Diese Bakterien des Standards wurden zu 16 verschiedenen Proben von jeweils 100 mg zugegeben. Bei den Proben handelt es sich um 15 Stuhlproben einer Maus und eine Kontrollprobe. Es wurde erwartet, dass 2x10⁸ (Spike 1), 2x10⁹ (Spike) und 2x10⁸ (Spike 3) 16S rDNA-Kopien pro Probe vorhanden sein sollten.

Nach der Entnahme der Stuhlproben erfolgte deren Lagerung bis zur Analyse bei -80 °C. Für die Analyse wurden 100 mg jeder Stuhlprobe entnommen und unter Zugabe von 180 µl Roche Bacteria Lysis Buffer in 250 µl PBS-Puffer resuspendiert. Anschließend erfolgte die Zugabe der Spike-Bakterien-Mischung in einem Volumen von 30 µl.

Die Prälyse der Mikrobiota (inkl. der zugesetzten Spike-Bakterien) erfolgte durch kombinierte enzymatische (durch Proteinase K) und mechanische Zelllyse (wiederholtes "Bead-Beating" mit Zirconia-Beads (d=0,1 mm) im Qiagen TissueLyser, 2x2 Min, 30 Hz).

Die DNA-Isolierung aus dem Lysat erfolgte unter Verwendung des MagNA Pure 96 DNA and Viral NA Large Volume Kits (Roche #06374891001) auf einem MagNA Pure 96 System (Roche) und Elution der DNA in 100 µl Elutionspuffer.

Als Spike-Bakterien wurden *Alicyclobacillus acidiphilus* ("Spike 1"; Phylum: Firmicutes, thermophil, Gram-positiv, Endosporen-Bildner, 6 16S rDNA-Kopien, 54 % G/C-Gehalt), *Salinibacter ruber* ("Spike 2"; Phylum: Bacteroidetes, halophil, Gram-negativ, 1 16S rDNA-Kopie, 66% G/C-Gehalt) und *Rhizobium radiobacter* ("Spike 3"; Phylum: Alpha-Proteobacteria, mesophil, Gram-negativ, 3 16S rDNA-Kopien, 59% G/C-Gehalt) verwendet. Diese Spike-Bakterien wurden ausgewählt, da sie sich insbesondere sowohl in genomisch kodierter 16S rRNA-Kopienzahl als auch in physiologischen Eigenschaften unterscheiden.

Die jeweiligen Spike-Bakterien wurden unter Verwendung geeigneter Nährmedien und Wachstumsbedingungen kultiviert. Die Bestimmung der Gesamtzellzahl der Bakterien erfolgte durch spektralphotometrische Messung der Optischen Dichte (OD₆₀₀ nm) im Nährmedium. Anschließend wurden die einzelnen Bakterien-Isolate auf die gewünschte Zellzahl/ml eingestellt. Zur Erzeugung der als Spike-Bakterien vorgesehenen Mischung wurden die drei Bakterienisolate in einem Gesamtvolumen von 30 µl mit jeweils 2x10⁸ Zellen/ml (A*. acidiphilus*), 3x10⁹ Zellen/ml (S. *ruber*) und 2x10⁸ Zellen/ml (*R*. *radiobacter*) gepoolt (bzw. vereinigt). Die Ergebnisse der Bestimmung der optischen Dichte sind für die drei ausgewählten Bakterienspezies in Tabelle 1 dargestellt.

**Tabelle 1:**

| **Bakterienspezies** | **OD1 = Zellen/ml** | **16S rDNA-Kopien/ Genom** | **OD_{600 nm}** | **Zellzahl pro ml** | **16S rDNA-Kopien pro Probe (Volumen 30 µl)** | **gewünschte 16 S Kopienzahl pro ml** |
|---|---|---|---|---|---|---|
| *A. acidiphilus* | 1,17x10⁹ | 6 | 33,4 | 3,91x10¹⁰ | 2,0x10⁸ | 6,67x10⁹ |
| *S. ruber* | 2,87x10⁸ | 1 | 401,2 | 1,15x10¹¹ | 3,0x10⁹ | 1,00x10¹¹ |
| *R. radiobacter* | 8,10x10⁸ | 3 | 75,2 | 6,09x10¹⁰ | 2,0x10⁸ | 6,67x10⁹ |

Wie oben erwähnt ist es möglich, eine quantitative Echtzeit-PCR der Proben durchzuführen, um eine Quantifizierung der Spike-Bakterien in der gesamten Mischung vorzunehmen. Figur 2 zeigt exemplarisch Amplifikationskurven der quantitativen real-time PCR zur Quantifizierung der 16S rDNA-Kopienzahl für die Proben 1 - 16 in grau (Kreissymbole für Messpunkte) anhand eines schwarz (Rechtecksymbole für Messpunkte) dargestellten externen Plasmid-Standards für Spike-Bakterium "Spike 1" (*Salinibacter ruber* (stellvertretend)). Dazu wurden 2 µl des wie oben beschrieben gewonnenen DNA-Extrakts zur Quantifizierung der Spike-Bakterien in einer quantitative Echtzeit-PCR unter Verwendung von speziespezifischen 16S rDNA-Primern, TaqMan-Sonden und Plasmid-Standards analysiert. Die PCR wurde auf einem Roche LightCycler 480 II unter Verwendung des LightCycler 480 Probes Master (Roche) durchgeführt.

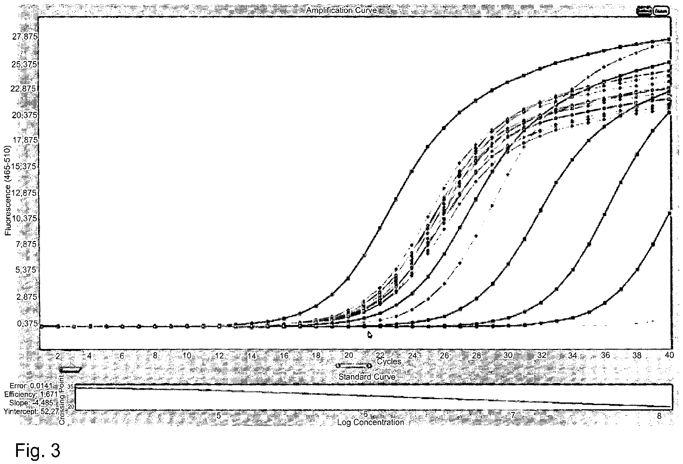

Figur 3 zeigt eine grafische Darstellung der durch quantitative Echtzeit-PCR bestimmten Kopienzahl von 16S rDNA. Die 16S rDNA-Kopienzahlen wurde durch quantitative real-time PCR für drei definierte Spike-Kontrollen (Spike 1 (*S. ruber,* schrafiert, unten), Spike 2 (*A*. a*cidiphilus,* grau, mittig) und Spike 3 (*R.radiobacter,* gepunktet, oben)) in den 16 DNA-Extrakten aus Stuhl-Proben zur Analyse des Darmmikrobioms der Maus bestimmt. Es ist zu erkennen, dass in allen Proben die Kopienzahl 16S rDNA von Spike 2 mit Werten zwischen 2,79x10⁹ und 4,48x10⁹ am größten ist und in allen Proben die Kopienzahl 16S rDNA von Spike 1 mit Werten zwischen 6,6x10⁷ und 1,42x10⁸ am geringsten ist. Als Standard wurde ein heterogener 16S rDNA Plasmidstandard verwendet. Probe 28 stellt eine Kontrollprobe dar und enthält ausschließlich eine Mischung der drei verwendeten Spike-Bakterien.

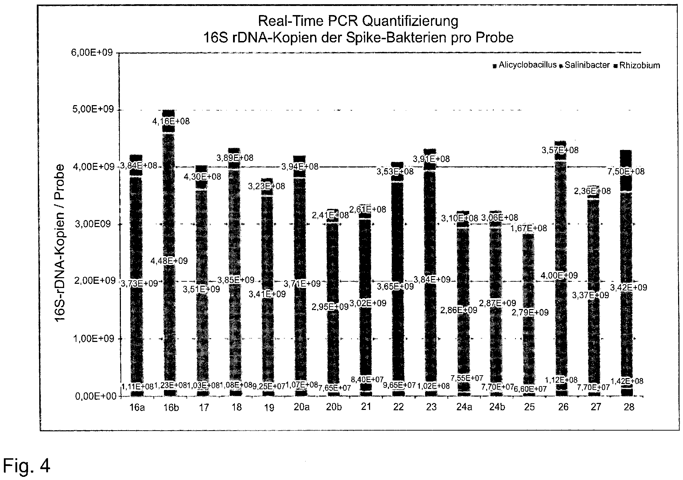

Figur 4 zeigt eine beispielhafte Amplifikationskurve einer quantitativen Echtzeit-PCR zur Quantifizierung der gesamten 16S rDNA-Kopienzahl nach Zusatz eines externen Standards unter Verwendung universeller Primer. Auch hier sind die Amplifikationskurven der quantitativen real-time PCR zur Quantifizierung der gesamten 16S rDNA-Kopienzahl für die Proben 1 - 16 (grau, Kreise) anhand eines externen Standards (schwarz, Rechtecke) dargestellt. Als Standard wurde stellvertretend Spike-Bakterium "Spike 1" verwendet. Die quantitative Echtzeit-PCR erfolgte unter Verwendung universeller 16S rDNA-Primern.

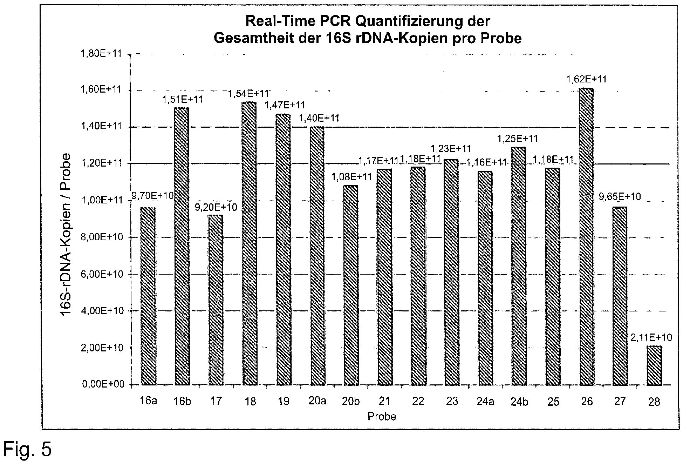

Figur 5 zeigt eine grafische Darstellung der durch quantitative Echtzeit-PCR bestimmten gesamten 16S rDNA-Kopienzahl. Die Gesamt 16S rDNA-Kopienzahlen in den 16 DNA-Extrakten aus Stuhl-Proben der Maus liegt dabei zwischen 9,2x10¹⁰ und 1,62x10¹¹. Bei der Probe 28 handelt es sich um eine Kontrollprobe, die ausschließlich eine Mischung aus den drei verwendete Spike-Bakterien enthält.

In den Stuhlproben 1 - 15 (jeweils 100 mg Stuhl) wurden durchschnittlich 1x10¹¹ 16S rDNA-Kopien quantitativ bestimmt. Zusammen mit der Quantifizierung von *S.ruber* 4x10⁹, *R. radiobacter* 4x10⁸ und *A. acidiphilus* 8x10₇ ergibt sich der Anteil der exogen zugegebenen Spike-Bakterien in der Mikrobiom-Probe zu etwa 4 % (Spike 1), 0,8 % (Spike 2) und 0,4 % (Spike 3) respektive.
die Zur PCR-Amplifikation der gesamten 16S-rDNA im DNA-Extrakt zur DNA-Sequenzierung werden 25 ng des wie oben beschrieben gewonnenen DNA-Eluats in einer konventionellen PCR mit den universellen Primern 341F/1061R amplifiziert. Die DNA-Konzentration und die Reinheit der isolierten DNA aus den Stuhlproben wurde mit dem NanoDrop 2000 UV/Vis Spektralphotometer (Thermo) bestimmt.

Die Ergebnisse der Bestimmung der DNA-Konzentration nach DNA-Isolierung der Stuhl-Lysate mit dem MagNA Pure 96 System (Roche) durch spektralphotometrische Messung ist in Tabelle 2 dargestellt

**Tabelle 2:**

| Sample ID | ng/ul | A₂₆₀ | A₂₈₀ | 260/280 | 260/230 |
|---|---|---|---|---|---|
| 16a | 345,41 | 6,908 | 3,309 | 2,09 | 2,27 |
| 16b | 368,48 | 7,37 | 3,539 | 2,08 | 2,24 |
| 17 | 370,56 | 7,411 | 3,571 | 2,08 | 2,22 |
| 18 | 363,99 | 7,28 | 3,491 | 2,09 | 2,27 |
| 19 | 395,28 | 7,906 | 3,806 | 2,08 | 2,26 |
| 20a | 452,35 | 9,047 | 4,371 | 2,07 | 2,3 |
| 20b | 460,17 | 9,203 | 4,444 | 2,07 | 2,29 |
| 21 | 266,06 | 5,321 | 2,534 | 2,1 | 2,25 |
| 22 | 292,55 | 5,851 | 2,84 | 2,06 | 2,21 |
| 23 | 361,42 | 7,228 | 3,503 | 2,06 | 2,28 |
| 24a | 423,92 | 8,478 | 4,134 | 2,05 | 2,25 |
| 24b | 407,93 | 8,159 | 3,964 | 2,06 | 2,21 |
| 25 | 400,12 | 8,002 | 3,883 | 2,06 | 2,28 |
| 26 | 478,44 | 9,569 | 4,632 | 2,07 | 2,29 |
| 27 | 409,27 | 8,185 | 3,963 | 2,07 | 2,21 |
| 28 | 129,45 | 2,589 | 1,248 | 2,08 | 2,15 |

Darin bedeutet A₂₆₀ die Absorption bei einer Wellenlänge von 260 nm und A₂₈₀ die Absorption bei einer Wellenlänge von 280 nm. 260/280 bzw. 260/230 gibt das Verhältnis der Absorption bei den jeweiligen Wellenlängen an.

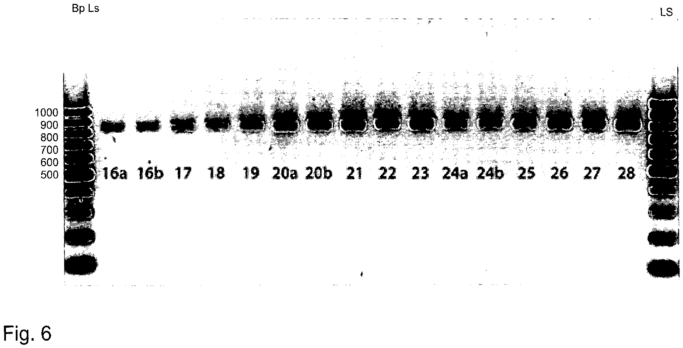

Während der Amplifikation des 800 Bp-DNA-Fragments werden außerdem die zur Sequenzierung mit dem GS Junior System notwendigen Titanium/Lib-L Adaptoren und die barcode-Sequenzen angefügt. Das Amplifikat wird nach der PCR gereinigt und mit Hilfe des KAPA Library Quant Kits (Kapa Biosystems) quantifiziert. Wie in Fig. 6 gezeigt ist, kann mittels Agarosegelelektrophorese (2,5 % (w/v) Agarose) der 16S rDNA-Amplifikate zur Mikrobiom-Analyse von 16 Stuhlproben gezeigt werden, dass diese die erwartete Fragmentgröße von etwa 800 Bp (Basenpaaren) aufweisen.

Zur Sequenzierung werden jeweils 1x10⁶ Kopien der 16 PCR-Amplifikate gemischt und mit dem GS Junior Plus System von Roche mit Hilfe des GS Junior+ emPCR Kits (Lib-L) und GS Junior+ Sequencing Kit XL+ analysiert.

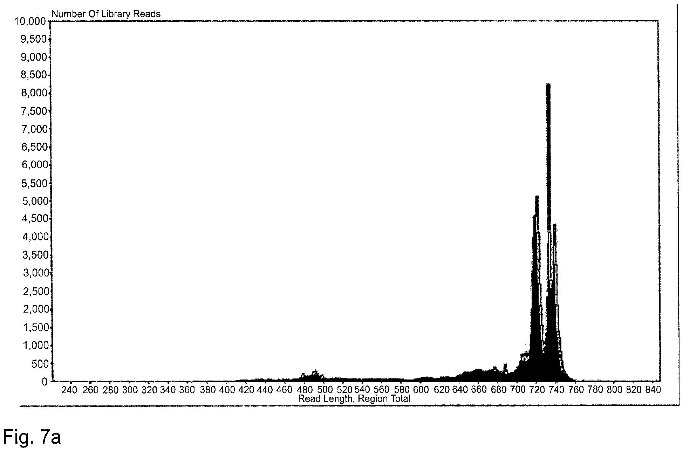

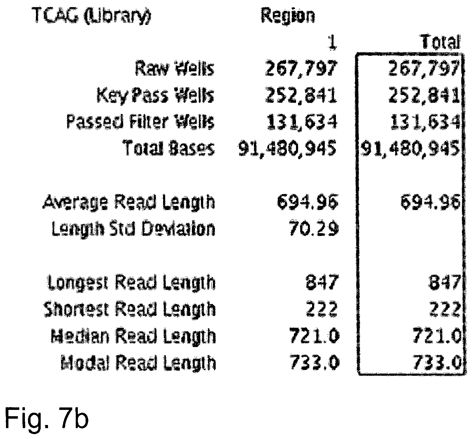

Die Analyse der Sequenz-Rohdaten wurde mittels der Roche/454 Analysis Software (Version 3.0) durchgeführt. Wie aus Fig. 7 ersichtlich ist, haben insgesamt 131.634 Reads der erwarteten mittleren Länge von etwa 720 Bp die Qualitätsfilter durchlaufen. Weiterhin zeigt Fig. 7 eine Darstellung der Sequenzierdaten in der Roche/454 Analysis Software (V 3.0). Die Größenverteilung der Reads zeigt eine mittlere Sequenzlänge von 721 Bp.

Zur weiteren Analyse, insbesondere zur Zuordnung der Reads zu den einzelnen Barcodes (Proben) und zur Durchführung weiter Qualitätskontrollschritten (Fehlerreduktion) wurde das frei verfügbare Softwarepaket Qiime (Version 1.8.0) verwendet. Von den ursprünglich 131.634 Reads wurden etwa 50.000 Reads entfernt, welche zuvor festgelegten Qualitätskriterien nicht erfüllten. Bei diesen Qualitätskriterien handelte es sich um Überschreitug der zugelassenen Anzahl an Homopolymeren, mehrere aufeinanderfolgende Basen mit schlechter Qualitätsbewertung und Primermismatch. Demnach standen 79.818 Reads für die weitere Analysen mit Hilfe der Qiime Pipeline zur Verfügung. Die Verteilung der Reads auf die einzelnen Proben ist in Tabelle 3 dargestellt. Die Tabelle 3 zeigt eine gleichmäßige Verteilung der Reads , wobei die aufsteigende Reihenfolge der MIDs 2 - 31 der Reihenfolge der Proben 16 - 28 entspricht.

**Tabelle 3:**

| Barcodes in mapping file | | | |
|---|---|---|---|
| Num Samples 16 | | | |
| Sample ct min/max/mean: 3749 / 6785 / 4988.62\| | | | |
| Sample | Sequence | Count | Barcode |
| MID5 | 6785 | ATCAGACACG | |
| MID2 | 6197 | ACGCTCGACA | |
| MID12 | 5889 | TACTGAGCTA | |
| MID31 | 5260 | AGCGTCGTCT | |
| MID11 | 5237 | TGATACGTCT | |
| MID13 | 5182 | CATAGTAGTG | |
| MID8 | 5018 | CTCGCGTGTC | |
| MID10 | 4858 | TCTCTATGCG | |
| MID14 | 4834 | CGAGAGATAC | |
| MID3 | 4627 | AGACGCACTC | |
| MID15 | 4582 | ATACGACGTA | |
| MID16 | 4554 | TCACGTACTA | |
| MID9 | 4546 | TAGTATCAGC | |
| MIDI | 4516 | ACGAGTGCGT | |
| MID7 | 3984 | CGTGCTCTCTA | |
| MID17 | 3749 | CGTCTAGTAC | |
| Totat | number segs written | | 79818 |

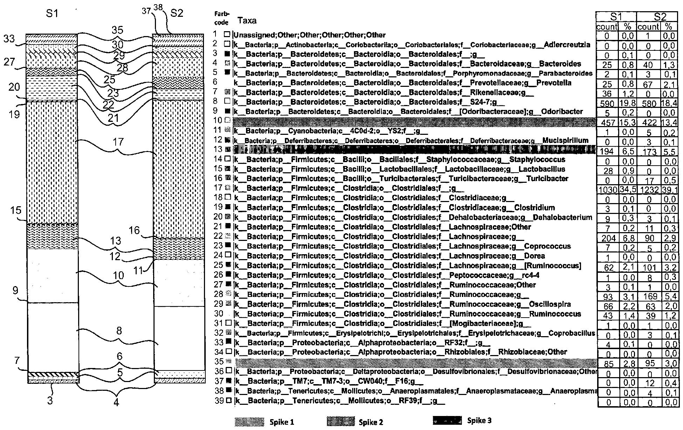

Zur weiteren Analyse wurden außerdem die DNA-Sequenzen die eine zuvor festgelegte Sequenzidentität übersteigen zu sogenannten OTUs (Operational Taxonomic Units) zusammengefasst bzw. geclustert. In dem in Fig 8 gezeigten Beispiel wurde die minimale Sequenzidentität auf > 97 % festgelegt. Durch Abgleich mit der Silva 16S rDNA Datenbank (release 111) wurden diese einer Taxonomie zugeordnet. Die daraus resultierende OTU-Tabelle (biom-Format Version 1.3.1), welche die absolute Anzahl an Reads für jedes zugeordnete OTU enthält, dient als Grundlage zur Darstellung der relativen Häufigkeiten einzelner Taxa sowie zur Normalisierung der Reads der Darm-Mikrobiota anhand der erzielten Reads für die zuvor zugegebenen Spike-Bakterien. In Figur 8 ist die relative Häufigkeit von taxonomisch annotierten OTUs in 454 GS Junior Sequenzdaten für zwei ausgewählte Proben beispielhaft dargestellt. Auf Basis dieser Daten ist sehr leicht ein Vergleich zwischen den relativen Häufigkeiten verschiedener Proben möglich. So können beispielsweise Veränderungen des Mikrobioms zwischen verschiedenen Zeitpunkten von Probeentnahmen detektiert werden.

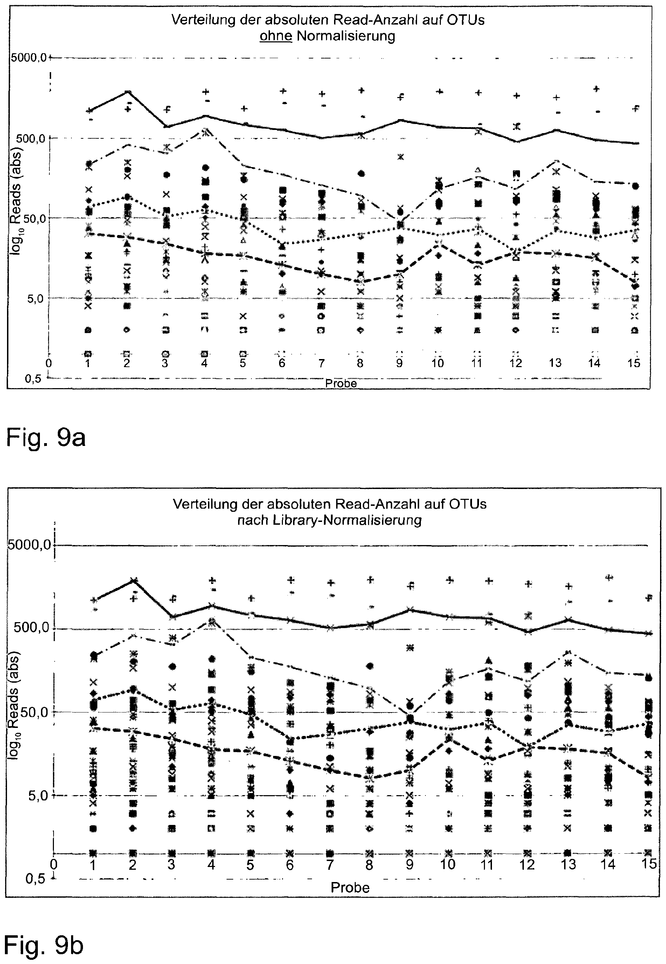

In den Figuren 9a - 9c ist die Verteilung der in der Mikrobiom-Analyse erhaltenen Reads je zugeordneter taxonomischen Einheiten (OTUs) für alle 15 analysierten Stuhl-Proben dargestellt. Die ausschließlich Spike-DNA enthaltende Kontrollprobe 16 ist zur besseren Übersichtlichkeit nicht dargestellt.

Die Figur 9a zeigt dabei die auf dem Genus-Level berechneten Read-Zahlen für die zugeordneten OTUs. Bereits ohne Normalisierung ist zu erkennen, dass die Readzahlen der für die einzelnen Spikes (*Salinibacter ruber* (durchgezogene Linie), *Rhizobium radiobacter* (gepunktete Linie) und *Alicyclobacillus acidiphilus* (gestrichelte Linie)) über alle Proben im Wesentlichen im selben Bereich liegen. Es ist demnach von einer vergleichbaren Probenbehandlung auszugehen. Zusätzlich wurde ein ausgewähltes OTU der Mikrobiota - *Ruminococcaceae* als strichpunktierte Linie hervorgehoben, welches die Veränderung der Read-Verteilung bereits auch ohne Normalisierung erkennen lässt. Die Schwankungen der Read-Zahlen schwanken über einen deutlich größeren Bereich als für die Spikes.

Noch deutlicher wird die Eignung der Spikes als Standards bei einer Normalisierung der Ergebnisse auf eine gleiche Anzahl Reads für jede Probe (Library Size Normalisierung), wie sie in Fig. 9b dargestellt ist. Auch hier ist *Salinibacter ruber* als durchgezogene Linie, *Rhizobium radiobacter* als gepunktete Linie und *Alicyclobacillus acidiphilus* als gestrichelte Linie hervorgehoben. Die Schwankungen der Read-Zahlen für das ausgewählte OTU der Mikrobiota - *Ruminococcaceae* welches erneut als strichpunktierte Linie hervorgehobenist, ist noch deutlicher zu erkennen.

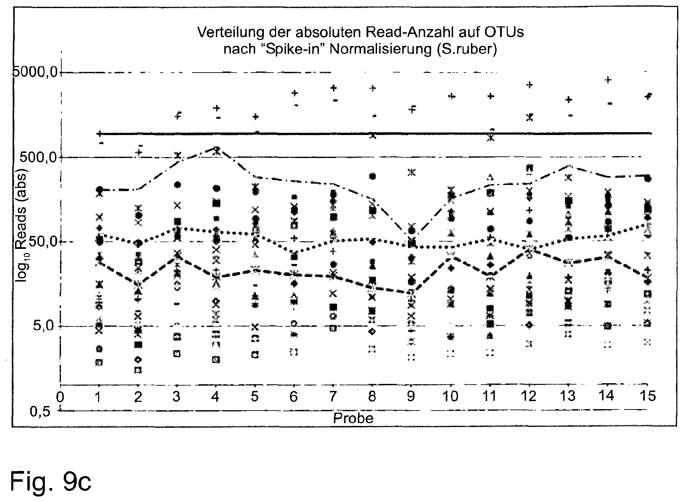

Figur 9c zeigt schließlich die Verteilung der Read-Zahlen nach Normalisierung auf die Read-Zahlen der für das Spike-Bakterium *S*.*ruber* erhaltenen Gesamt-Reads. Die Spike-In Normalisierung (auf *S.ruber*) zeigt anschaulich eine gleichmäßige Verteilung der bekannten Read-Anzahl für die beiden weiteren zugegeben Spike-Bakterien (*A*. *acidiphilus* und *R. radiobacter*)*.* Die Genauigkeit der Messwerte bei nur wenigen Reads pro annotiertem OTU einer Probe nimmt ab (Poisson-Verteilung), weshalb die Schwankung der Werte bei dem ebenfalls in gleicher Menge pro Probe zugegebenen Spike-Bakterium *A.acidiphiilus* auch nach Normalisierung noch höher sind, als bei dem Spike-Bakterium *R*. *radiobacter.* Dies belegt, dass die Zugabe der unterschiedlichen Spike-Bakterien in verschiedenen Konzentrationsbereichen insbesondere deshalb sinnvoll, da die Zusammensetzung des Mikrobioms vor Beginn des Analyse-Prozesses nicht exakt vorhergesagt werden kann. Für das Spike-Bakterium *R*. *radiobacter,* welches jeweils in gleicher Menge zu 2x10⁸ Zellen/ml zu den Proben gegeben war, zeigt sich erst durch Normalisierung auf die zum Spike-Bakterium *S.ruber* zugeordneten Reads eine gleichmäßige Verteilung der Reads auf die Proben. Eine derartige Darstellung ermöglicht es, Varianzen einzelner OTUs gegenüber einem (oder mehreren) Standard(s) aufzuzeigen und sogar zu quantifizieren. Im gezeigten Beispiel sind die Varianzen zwischen den einzelnen Proben am Beispiel des OTUs der Mikrobiota - *Ruminococcaceae* (als strichpunktierte Linie) dargestellt. Anhand dieser Daten wäre eine quantitative Auswertung möglich, die insbesondere die starken Veränderungen in den Proben 4 (Maximum) und 9 (Minimum) erfassen könnte. Analog kann auch für andere OTUs, die beispielsweise mit einem Krankheitsbild in Verbindung gebracht werden können, eine Abweichung zwischen verschiedenen Proben detektiert werden.

Die in den Fig. 9a - 9c genutzten Zeichen für einzelne OTUs könnten der Tabelle 4 entnommen werden.

**Tabelle 4**

| |
|---|
| ◆ Bacteria;_Actinobacteria;_Coriobacteriia;_Coriobacteriales;_Coriobacteriaceae;_Enterorhabdus |
| ■ Bacteria:_Bacteroidetes;_Bacteroidia;_Bacteroidales;_Bacteroidaceae;_Bacteroides |
| ▲ Bacteria;_Bacteroidetes;_Bacteroidia;_Bacteroidales;_Porphyromonadaceae;_Odoribacter |
| × Bacteria;_Bacteroidetes;_Bacteroidia;_Bacteroidales;_Porphyromonadaceae;_Parabacteroides |
| 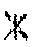 Bacteria;_Bacteroidetes;_Bacteroidia;_Bacteroidales;_Prevotellaceae;_uncultured |
| ● Bacteria;_Bacteroidetes;_Bacteroidia;_Bacteroidales;_Rikenellaceae;_Alistipes |
| + Bacteria;_Bacteroidetes;_Bacteroidia;_Bacteroidales;_Rikenellaceae;_RC9_gut_group |
| - Bacteria;_Bacteroidetes;_Bacteroidia;_Bacteroidales;_S24-7;_uncultured_bacterium |
| 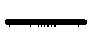 Bacteria:_Bacteroidetes;_Cytophagia;_Order_II_Incertae_Sedis;_Rhodothermaceae;_Salinibacter |
| ◆ Bacteria;_Bacteroidetes;_VC2,1_Bac22;_uncultured_bacterium;Other;Other |
| ■ Bacteria;_Candidate_division_TM7;_uncultured_bacterium;Other;Other;Other |
| ▲ Bacteria;_Cyanobacteria;_4COd-2;_uncultured_bacterium;Other,Other |
| × Bacteria;_Deferribacteres;_Deferribacterales;_Deferribacteraceae;_Mucispirllium;_uncultured_bacterium |
| 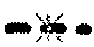 Bacteria;_Firmicutes;_Bacilli;_Bacillales;_Alicyclobacillaceae;_Alicyclobacillus |
| ● Bacteria;_Firmicutes;_Bacilli;_Bacillales;_Staphylococcaceae;_Staphylococcus |
| + Bacteria;_Firmicutes;_Bacilli;_Lactobacillales;_Lactobacillaceae;_Lactobacillus |
| - Bacteria;_Firmicutes;_Clostridia;_Clostridiales;_Christensenellaceae;_Christensenella |
| - Bacteria;_Firmicutes;_Clostridia;_Clostridiales;_Christensenellaceae;_uncultured 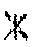 |
| ◆ Bacteria;_Firmicutes;_Clostridia;_Clostridiales;_Clostridiaceae;_Candidatus_Arthromitus |
| ■ Bacteria;_Firmicutes;_Clostridia;_Clostridiales;_Eubacteriaceae;_Anaerofustis |
| ▲ Bacteria;_Firmicutes;_Clostridia;_Clostridiales;_Family_XIII_Incertae_Sedis;_Anaerovorax_ |
| × Bacteria;_Firmicutes;_Clostridia;_Clostridiales;_Family_XIII_Incertae_Sedis:_Incertae_Sedis |
| Bacteria;_Firmicutes;_Clostridia;_Clostridiales;_Family_XIII_Incertae_Sedis;_uncultured |
| ○ Bacteria;_Firmicutes;_Clostridia;_Clostridiales;_Lachnospiraceae;_Acetitomaculum |
| + Bacteria;_Firmicutes;_Clostridia;_Clostridiales;_Lachnospiraceae;_Anaerostipes |
| - Bacteria;_Firmicutes;_Clostridia;_Clostridiales;_Lachnospiraceae;_Blautia |
| - Bacteria;_Firmicutes;_Clostridia;_Clostridiales;_Lachnospiraceae;_Coprococcus |
| ◆ Bacteria;_Firmicutes;_Clostridia;_Clostridiales;_Lachnospiraceae;_Dorea |
| ■ Bacteria;_Firmicutes;_Clostridia;_Clostridiales;_Lachnospiraceae;_Incertae_Sedis |
| ▲ Bacteria;_Firmicutes;_Clostridia;_Clostridiales;_Lachnospiraceae;_Marvinbryantia |
| × Bacteria;_Firmicutes;_Clostridia;_Clostridiales;_Lachnospiraceae;_Moryella |
| 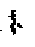 Bacteria;_Firmicutes;_Clostridia;_Clostridiales;_Lachnospiraceae;_Pseudobutyrivibrio |
| ● Bacteria;_Firmicutes;_Clostridia;_Clostridiales;_Lachnospiraceae;_Roseburia |
| + Bacteria;_Firmicutes;_Clostridia;_Clostridiales;_Lachnospiraceae;_uncultured |
| - Bacteria;_Firmicutes;_Clostridia;_Clostridiales;_Lachnospiraceae;_uncultured_bacterium |
| - Bacteria;_Firmicutes;_Clostridia;_Clostridiales;_Peptococcaceae;_Peptococcus |
| ◆ Bacteria;_Firmicutes;_Clostridia;_Clostridiales;_Peptococcaceae;_uncultured |
| □ Bacteria;_Firmicutes;_Clostridia;_Clostridiales;_Peptostreptococcaceae;_Incertae_Sedis |
| ▲ Bacteria;_Firmicutes;_Clostridia;_Clostridiales;_Ruminococcaceae:_Anaerotruncus |
| × Bacteria;_Firmicutes;_Clostridia;_Clostridiales;_Ruminococcaceae;_Incertae_Sedis |
| 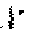 Bacteria;_Firmicutes;_Clostridia;_Clostridiales;_Ruminococcaceae;_Oscillibacter |
| 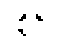 Bacteria;_Firmicutes;_Clostridia;_Clostridiales;_Ruminococcaceae;_Oscillospira |
| + Bacteria;_Firmicutes;_Clostridia;_Clostridiales;_Ruminococcaceae;_Ruminococcus |
| - Bacteria;_Firmicutes;_Clostridia;_Clostridiales;_Ruminococcaceae;_Subdoligranulum |
| 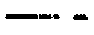 -Bacteria;_Firmicutes;_Clostridia;_Clostridiales;_Ruminococcaceae;_uncultured |
| ◆ Bacteria;_Firmicutes;_Clostridia;_Clostridiales;_uncultured;_uncultured_bacterium |
| □ Bacteria;_Firmicutes;_Erysipelotrichi;_Erysipelotrichales;_Erysipelotrichaceae;_Allobaculum |
| 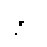 Bacteria;_Firmicutes;_Erysipelotrichi;_Erysipelotrichales;_Erysipelotrichaceae;_Incertae_Sedis |
| × Bacteria;_Firmicutes;_Erysipelotrichi;_Erysipelotrichales;_Erysipelotrichaceae;_Turicibacter |
| 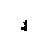 Bacteria;_Firmicutes;_Erysipelotrichi;_Erysipelotrichales;_Erysipelotrichaceae;_uncultured |
| ○ Bacteria:_Firmicutes;_Erysipeilorichi;_Erysipelotrichales;_Erysipelotrichaceae;_uncultured_bacterium |
| † Bacteria;_Proteobacteria;_Alphaproteobacteria;_Rhizobiales;_Phyllobacteriaceae;_uncultured |
| •••• Bacteria;_Proteobacteria;_Alphaproteobacteria;_Rhizobiales;_Rhizobiaceae;_Rhizobium |
| Bacteria;_Proteobacteria;_Alphaproteobacteria;_Rhodospiritiales;_Rhodospirillaceae;_Thalassospira |
| ◆ Bacteria;_Proteobacteria;_Betaproteobacteria;_Burkholderiales;_Alcaligenaceae;_Parasutterella |
| 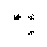 Bacteria;_Proteobacteria;_Deltaproteobacteria;_Desulfovibrionales;_Desulfovibrionaceae;_Bilophila |
| △ Bacteria;_Tenericutes;_Mollicutes;_Anaeroplasmatales;_Anaeroplasmataceae;_Anaeroplasma |
| ^ Bacteria;_Tenericutes;_Mollicutes;_RF9;_uncultured_bacterium;Other |

## Patentansprüche

1. Verfahren zur Mikrobiom-Analyse,
**dadurch gekennzeichnet, dass**
einer Probe ein quantifizierbarer Standard zugesetzt wird, der mindestens ein Bakterien-Isolat umfasst, das mindestens zwei Bakterienspezies umfasst, und die Probe anschließend gemeinsam mit dem Standard analysiert wird, wobei die Mikrobiomanalyse mindestens die Schritte
- Amplifikation von universellen Markergenen,
- Nukleinsäuresequenzierung,
- Sequenzanalyse und
- mindestens einen Quantifizierungsschritt mittels quantitativer Echtzeit-PCR umfasst.

2. Verfahren nach Anspruch 1,
**dadurch gekennzeichnet, dass**
die Mikrobiomanalyse weiterhin Schritte beinhaltet, die ausgewählt sind aus der Gruppe bestehend aus Probenentnahme, DNA-Isolierung, Qualitätskontrolle, Clustern von ähnlichen Sequenzen, taxonomische Annotation, statistische Analyse und Interpretation der ermittelten Daten.

3. Verfahren nach Anspruch einem der vorangegangenen Ansprüche,
**dadurch gekennzeichnet, dass**
der quantifizierbare Standard der Probe vor der DNA-Isolierung zugesetzt wird.

4. Verfahren nach Anspruch einem der vorangegangenen Ansprüche,
**dadurch gekennzeichnet, dass**
durch Auswahl universeller PCR-Primer Markergen-Regionen der gesamten Mikrobiota der Mischung aus Probe und Standard mittels quantitativer Echtzeit-PCR amplifiziert werden und bevorzugt anschließend die Kopienzahl der Markergene im resultierenden PCR-Amplifikationsgemisch, und besonders bevorzugt auch in der ursprünglichen Probe nach der Isolierung der DNA, quantitativ bestimmt wird.

5. Verfahren nach Anspruch einem der vorangegangenen Ansprüche,
**dadurch gekennzeichnet, dass**
einer quantitativer Echtzeit-PCR ein externer Quantifizierungsstandard und universelle Primerpaare zugesetzt werden und die Gesamtzahl an Markergenkopien in der Mischung aus Probe und Standards durch absolute Quantifizierung bestimmt wird.

6. Verfahren nach Anspruch einem der vorangegangenen Ansprüche,
**dadurch gekennzeichnet, dass**
der Standard aus einem oder mehreren Bakterien-Isolaten mindestens 3, weiter bevorzugt mindestens 5, weiter bevorzugt mindestens 10 verschiedene Bakterienspezies umfasst.

7. Verfahren nach einem der vorangegangenen Ansprüche,
**dadurch gekennzeichnet, dass**
mindestens eine Bakterienspezies eines Bakterien-Isolats mindestens 2, bevorzugt mindestens 3, bevorzugt mindestens 4, weiter bevorzugt mindestens 5, weiter bevorzugt mindestens 6, besonders bevorzugt alle der Eigenschaften aus einer Gruppe von Eigenschaften aufweist, die die Eigenschaften
- Nukleinsäuresequenzabschnitte eines Markergens der Bakterienspezies weisen weniger als 95 % Sequenzidentität mit den in verfügbaren Humanmikrobiom-Sequenzdatenbanken hinterlegten Nukleinsäuresequenzen auf,
- die Bakterienspezies gehört nicht einem Genus an, dessen Vorkommen im Human- und/oder Tier-Mikrobiom beschrieben ist,
- die Bakterienspezies, bevorzugt die Spike-Bakterien, ist/sind unter verfügbaren mikrobiologischen Methoden im Labor kultivierbar,
- die Bakterienspezies, bevorzugt die Spike-Bakterien, ist/sind mit mikroskopischen Methoden in wässriger Lösung quantifizierbar,
- die Speziesmarker-Gen Kopien der Bakterienspezies der Spike-Bakterien sind durch eine spezifische quantitative Echtzeit-PCR quantifizierbar, bevorzugt auch in komplexen Gengemischen, besonders bevorzug auch in komplexen Spike-Bakterien-Mischungen,
- die Bakterienspezies gehört einem Phylum an, der im humanen Mikrobiom ebenfalls vertreten ist, wobei bei mehreren Bakterienspezies der Spike-Bakterien mindestens zwei der Bakterienspezies zu unterschiedlichen Phyla gehören,
- die intragenomische Anzahl der Speziesmarker-Gene der Bakterien der Spike-Bakterien ist bekannt und kann beim Prozess der Quantifizierung berücksichtigt werden, umfasst, und falls das Bakterien-Isolat mehrere Bakterienspezies umfasst mindestens zwei Bakterienspezies unterschiedliche Zellwandstrukturen besitzen und/oder mindestens zwei Bakterienspezies auswählbare Speziesmarker-Gene mit unterschiedlichem G/C-Gehalt aufweisen.

8. Verfahren nach Anspruch einem der vorangegangenen Ansprüche,
**dadurch gekennzeichnet, dass**
mindestens eine Bakterienspezies des Bakterien-Isolats, bevorzugt mindestens zwei Bakterienspezies des Bakterien-Isolats, weiter bevorzugt mindestens drei Bakterienspezies des Bakterien-Isolats, besonders bevorzugt mindestens fünf Bakterienspezies des Bakterien-Isolats aus einer Gruppe ausgewählt sind, die die Bakterienspezies *Salinibacter ruber, Alicyclobacillus acidiphilus, Rhizobium radiobacter, Sphaerobacter thermophilus, Thermomicrobium roseum, Thiomicrospira arctica, Acidimicrobium ferrooxidans, Alcanivorax borkumensis, Colwellia psychrerythraea, Idiomarina loihiensis, Marinilabilia saimonicolor, Thioalkalibacter halophilus, Rhodobaca bogoriensis, Sulfurimonas autotrophica, Arcobacter halophilus, Aquisalibacillus elongates, Haloactinobacterium album, Geobacillus stearothermophilus* und *Geobacillus caldoxylolyticus* umfasst.

9. Verfahren nach Anspruch einem der vorangegangenen Ansprüche,
**dadurch gekennzeichnet, dass**
eine Normalisierung der absoluten Menge an DNA-Sequenzen eines Clusters ähnlicher Sequenzen (OTUs) anhand der Read-Anzahl an Sequenzen eines oder mehrere Bakterien des Standards erfolgt.

10. Verwendung eines Kits zur Durchführung eines Verfahrens zur Mikrobiom-Analyse, gemäß einem der vorangegangenen Ansprüche,
**dadurch gekennzeichnet, dass**
das Kit einen quantifizierbaren Standard für eine Mikrobiom-Analyse, der mindestens ein Bakterien-Isolat umfasst, das mindestens zwei Bakterienspezies umfasst, und eine Anweisung zur Durchführung einer Mikrobiom-Analyse umfasst.

## Claims

1. Method for microbiome analysis,
**characterised in that**
a quantifiable standard is added to a sample, which standard comprises at least one bacterial isolate comprising at least two bacterial species, and the sample is subsequently analysed together with the standard, the microbiome analysis comprising at least the steps of
- amplifying universal marker genes,
- nucleic acid sequencing,
- performing sequence analysis, and
- at least one quantification step by quantitative real-time PCR.

2. Method according to claim 1,
**characterised in that**
the microbiome analysis also includes steps selected from the group consisting of sampling, isolating DNA, performing quality control, clustering similar sequences, performing taxonomic annotation, performing statistical analysis, and interpreting the recorded data.

3. Method according to any of the preceding claims,
**characterised in that**
the quantifiable standard is added to the sample before the DNA isolation.

4. Method according to any of the preceding claims,
**characterised in that**
by selecting universal PCR primers, marker gene regions in the entire microbiota of the mixture consisting of the sample and the standard are amplified by quantitative real-time PCR, and subsequently preferably the number of copies of the marker genes in the resulting PCR amplification mixture, and particularly preferably also in the original sample after the isolation of the DNA, is quantitatively determined.

5. Method according to any of the preceding claims,
**characterised in that**
an external quantification standard and universal primer pairs are added to a quantitative real-time PCR, and the total number of marker gene copies in the mixture consisting of the sample and the standard is determined by absolute quantification.

6. Method according to any of the preceding claims,
**characterised in that**
the standard comprises, from one or more bacterial isolates, at least 3, more preferably at least 5, more preferably at least 10 different bacterial species.

7. Method according to any of the preceding claims,
**characterised in that**
at least one bacterial species in a bacterial isolate comprises at least 2, preferably at least 3, preferably at least 4, more preferably at least 5, more preferably at least 6, particularly preferably all the properties from a group of properties which comprises
- nucleic acid sequence portions of a marker gene of the bacterial species have less than 95% sequence identity with the nucleic acid sequences stored in available human microbiome sequence databases,
- the bacterial species does not belong to a genus of which the existence has been described in the human and/or animal microbiome,
- the bacterial species, preferably the spike bacteria, can be cultivated in a laboratory using available microbiological methods,
- the bacterial species, preferably the spike bacteria, can be quantified in an aqueous solution using microscopic methods,
- the species marker gene copies of the bacterial species of the spike bacteria can be quantified by a specific quantitative real-time PCR, preferably also in complex gene mixtures, particularly preferably also in complex spike bacteria mixtures,
- the bacterial species belongs to a phylum that is also represented in the human microbiome, at least two of the bacterial species belonging to different phyla if there is a plurality of bacterial species of the spike bacteria,
- the intragenomic number of species marker genes of the bacteria of the spike bacteria is known and can be taken into account during the process of quantification, and if the bacterial isolate comprises a plurality of bacterial species, at least two bacterial species have different cell wall structures and/or at least two bacterial species have selectable species marker genes having different G/C contents.

8. Method according to any of the preceding claims,
**characterised in that**
at least one bacterial species in the bacterial isolate, preferably at least two bacterial species in the bacterial isolate, more preferably at least three bacterial species in the bacterial isolate, particularly preferably at least five bacterial species in the bacterial isolate, is/are selected from a group comprising *Salinibacter ruber, Alicyclobacillus acidiphilus, Rhizobium radiobacter, Sphaerobacter thermophilus, Thermomicrobium roseum, Thiomicrospira arctica, Acidimicrobium ferrooxidans, Alcanivorax borkumensis, Colwellia psychrerythraea, Idiomarina loihiensis, Marinilabilia salmonicolor, Thioalkalibacter halophilus, Rhodobaca bogoriensis, Sulfurimonas autotrophica, Arcobacter halophilus, Aquisalibacillus elongates, Haloactinobacterium album, Geobacillus stearothermophilus* and *Geobacillus caldoxylolyticus.*

9. Method according to any of the preceding claims,
**characterised in that**
the absolute quantity of DNA sequences of a cluster of similar sequences (OTUs) is normalised by the read number of sequences of one or more bacteria in the standard.

10. Use of a kit for carrying out a method for microbiome analysis, according to any of the preceding claims,
**characterised in that**
the kit comprises a quantifiable standard for microbiome analysis, which comprises at least one bacterial isolate comprising at least two bacterial species, and comprises instructions for carrying out microbiome analysis.

## Revendications

1. Procédé d'analyse du microbiome,
**caractérisé par le fait que**
à un échantillon est ajouté un étalon quantifiable qui comporte au moins un isolat bactérien qui comporte au moins deux espèces bactériennes, et l'échantillon est ensuite analysé conjointement avec l'étalon, l'analyse du microbiome comportant au moins les étapes :
- amplification de gènes marqueurs universels ;
- séquençage d'acides nucléiques ;
- analyse des séquences ; et
- au moins une étape de quantification au moyen d'une PCR quantitative en temps réel.

2. Procédé selon la revendication 1,
**caractérisé par le fait que**
l'analyse du microbiome inclut en outre des étapes qui sont choisies dans le groupe consistant en l'échantillonnage, l'isolement d'ADN, le contrôle de la qualité, le regroupement de séquences similaires, l'annotation taxonomique, l'analyse statistique et l'interprétation des données établies.

3. Procédé selon l'une des revendications précédentes,
**caractérisé par le fait que**
l'étalon quantifiable est ajouté à l'échantillon avant l'isolement d'ADN.

4. Procédé selon l'une des revendications précédentes,
**caractérisé par le fait que**
par sélection d'une amorce universelle de PCR des régions de gènes marqueurs du microbiote total du mélange d'échantillon et d'étalon sont amplifiées au moyen d'une PCR quantitative en temps réel, et de préférence ensuite le nombre de copies des gènes marqueurs dans le mélange d'amplification par PCR résultant, et de manière particulièrement préférée également dans l'échantillon initial après l'isolement de l'ADN, est déterminé de manière quantitative.

5. Procédé selon l'une des revendications précédentes,
**caractérisé par le fait que**
un étalon de quantification externe et des paires d'amorces universelles sont ajoutés à une PCR quantitative en temps réel et le nombre total de copies de gènes marqueurs dans le mélange d'échantillon et d'étalon est déterminé par quantification absolue.

6. Procédé selon l'une des revendications précédentes,
**caractérisé par le fait que**
l'étalon d'un ou plusieurs isolats bactériens comporte au moins 3, de manière davantage préférée au moins 5, de manière davantage préférée au moins 10 espèces bactériennes différentes.

7. Procédé selon l'une des revendications précédentes,
**caractérisé par le fait que**
au moins une espèce bactérienne d'un isolat bactérien présente au moins 2, de préférence au moins 3, de préférence au moins 4, de manière davantage préférée au moins 5, de manière davantage préférée au moins 6, de manière particulièrement préférée toutes les propriétés d'un groupe de propriétés, qui comporte les propriétés :
- des sections de séquences d'acides nucléiques d'un gène marqueur de l'espèce bactérienne présentent moins de 95 % d'identité de séquence avec les séquences d'acides nucléiques déposées dans des banques de données de séquences du microbiome humain disponibles ;
- l'espèce bactérienne n'appartient pas à un genre dont la présence est décrite dans le microbiome humain et/ou animal ;
- l'espèce bactérienne, de préférence les bactéries Spike, est/sont aptes à être cultivées au laboratoire par des méthodes microbiologiques disponibles ;
- l'espèce bactérienne, de préférence les bactéries Spike, est/sont quantifiables en solution aqueuse par des méthodes microscopiques ;
- les copies de gène marqueur d'espèce des espèces bactériennes des bactéries Spike sont quantifiables par une PCR quantitative en temps réel spécifique, de préférence également dans des mélanges de gènes complexes, de manière particulièrement préférée également dans des mélanges de bactéries Spike complexes ;
- l'espèce bactérienne appartient à un Phylum qui est représenté également dans le microbiome humain, où au moins deux des espèces bactériennes appartiennent à des Phylums différents dans le cas de plusieurs espèces bactériennes des bactéries Spike ;
- le nombre intragénomique des gènes marqueurs d'espèces des bactéries des bactéries Spike est connu et peut être pris en considération dans le cas du procédé de quantification,et si l'isolat bactérien comporte plusieurs espèces bactériennes, au moins deux espèces bactériennes possèdent des structures de paroi cellulaire différentes et/ou au moins deux espèces bactériennes présentent des gènes marqueurs d'espèces sélectionnables avec une teneur en G/C différente.

8. Procédé selon l'une des revendications précédentes,
**caractérisé par le fait que**
au moins une espèce bactérienne de l'isolat bactérien, de préférence au moins deux espèces bactériennes de l'isolat bactérien, de manière davantage préférée au moins trois espèces bactériennes de l'isolat bactérien, de manière particulièrement préférée au moins cinq espèces bactériennes de l'isolat bactérien sont choisies dans un groupe qui comporte les espèces bactériennes *Salinibacter ruber, Alicyclobacillus acidiphilus, Rhizobium radiobacter, Sphaerobacter thermophilus, Thermomicrobium roseum, Thiomicrospira arctica, Acidimicrobium ferrooxidans, Alcanivorax borkumensis, Colwellia psychrerythraea, Idiomarina loihiensis, Marinilabilia salmonicolor, Thioalkalibacter halophilus, Rhodobaca bogoriensis, Sulfurimonas autotrophica, Arcobacter halophilus, Aquisalibacillus elongates, Haloactinobacterium album, Geobacillus stearothermophilus* et *Geobacillus caldoxylolyticus.*

9. Procédé selon l'une des revendications précédentes,
**caractérisé par le fait que**
une normalisation de la quantité absolue de séquences d'ADN d'un cluster de séquences similaires (OTU) a lieu sur la base du nombre de lectures de séquences d'une ou plusieurs bactéries de l'étalon.

10. Utilisation d'un kit pour la réalisation d'un procédé d'analyse du microbiome, selon l'une des revendications précédentes,
**caractérisée par le fait que**
le kit comporte un étalon quantifiable pour une analyse du microbiome, qui comporte au moins un isolat bactérien qui comporte au moins deux espèces bactériennes, et des instructions pour la réalisation d'une analyse du microbiome.
